(19) Europäisches Patentamt
European Patent Office
Office européen des brevets

(11) EP 2 654 902 B1

(12) EUROPEAN PATENT SPECIFICATION

(45) Date of publication and mention
of the grant of the patent:
**06.03.2019 Bulletin 2019/10**

(51) Int Cl.:
*A61Q 11/00* (2006.01)     *A61K 8/24* (2006.01)
*A61K 8/86* (2006.01)     *A61K 8/04* (2006.01)
*A61K 8/20* (2006.01)

(21) Application number: **11850380.4**

(22) Date of filing: **09.06.2011**

(86) International application number:
**PCT/US2011/039823**

(87) International publication number:
**WO 2012/087374 (28.06.2012 Gazette 2012/26)**

(54) **COMPOSITION AND METHOD TO FLAVOR ORAL CARE COMPOSITIONS CONTAINING A CHLORINE DIOXIDE SOURCE**

ZUSAMMENSETZUNG UND VERFAHREN FÜR AROMATISCHE MUNDPFLEGEZUSAMMENSETZUNGEN MIT EINER CHLORDIOXIDQUELLE

COMPOSITION ET PROCÉDÉ POUR AROMATISER DES COMPOSITIONS DE SOIN BUCCAL CONTENANT UNE SOURCE DE DIOXYDE DE CHLORE

(84) Designated Contracting States:
**AL AT BE BG CH CY CZ DE DK EE ES FI FR GR HR HU IE IS IT LI LT LU LV MC MK MT NL NO PL PT RO RS SE SI SK SM TR**

(30) Priority: **25.05.2011 US 201113115815**
**22.12.2010 US 201061426279 P**

(43) Date of publication of application:
**30.10.2013 Bulletin 2013/44**

(73) Proprietor: **Micropure, INC.**
**Scottsdale, AZ 85260 (US)**

(72) Inventors:
• **RATCLIFF, James, L.**
**Pueblo West**
**CO 81007 (US)**
• **BLACK, Karen**
**Peoria**
**AZ 85345 (US)**
• **COOLEY, William, E.**
**Wyoming**
**OH 45215 (US)**
• **GARCIA, Esmeralda, Ann**
**Scottsdale**
**AZ 85254 (US)**

(74) Representative: **Tomkins & Co**
**5 Dartmouth Road**
**Dublin 6 (IE)**

(56) References cited:
**EP-A1- 0 613 678     US-A1- 2003 129 144**
**US-A1- 2003 129 144     US-A1- 2010 221 198**
**US-B1- 6 280 775**

**Description**

BACKGROUND OF THE INVENTION

1. Field of the Invention

The composition, either for humans or pets, may be in the form of a rinse, paste, gel, varnish, powder, impregnated floss, dissolving tablet or similar, incorporating: 1) chlorine dioxide source, 2) phosphate buffer, 3) flavoring system containing a polyoxyethylene sorbitan ester (emulsifier/suspender) and flavoring agent, 4) sweetener, and 5) water. The method emulsifies a flavoring agent in a polyoxythylene sorbitan ester to form a flavoring system, which when added to aqueous phosphate-buffered composition containing a chlorine dioxide source, results in stable, homogeneous flavored oral care compositions.

2. Description of Related Prior Art

**Chlorite Ion Compositions, Stabilized Chlorine Dioxide (SCD) Compositions, and Conditions/Diseases of the Oral Cavity**

In US 4330531, Alliger teaches dual phase germ-killing compositions comprised of sodium chlorite (first phase) and lactic acid (second phase) at pH less than 7. These compositions are intended for cleaning, sanitizing, deodorizing and disinfecting surface materials. The sodium chlorite is believed to be acidified by the acid to produce chlorine dioxide; and it specifically states that "most flavoring and coloring agents, for example, will react with chlorine dioxide." US 5616347 and US 6039934, by Alliger, further teach methods for dual-phase compositions which generate chlorine dioxide through the activation of chlorite, and both patents specifically include the use of glycerin as an irritation reducing compound or an additive.

In US 4861514, Hutchings teaches chlorine dioxide-containing compositions comprising sodium chlorite, water, and an initiator, with the compositions maintaining a viscosity appropriate for "suspendably retaining the gaseous chlorine dioxide formed subsequent to composition preparation." The initiator could be one or all of the following: materials to thicken aqueous compositions, dyes, materials including an aldehyde or an actual substituent group including perfumes containing such groups, perfumes not including an aldehyde substituent group, and reducing sugars. The patent specifically states that "it is believed the initiator interacts with chlorite in the aqueous composition to provide the chlorine dioxide, which interaction apparently ceases when an equilibrium concentration for the chlorine dioxide is reached." It states the interaction with the initiator to generate chlorine dioxide takes several days to occur; and that the greater the chlorite concentration, the longer time required for interaction. Thus Hutchings teaches that there can be an equilibrium between a chlorite reservoir and free chlorine dioxide gas which is moderated by a medium of varying viscosity or presence of aldehyde or sugar functional groups or perfumes. The present invention teaches that such an equilibrium for the purpose of maintaining active amounts of free therapeutic chlorine dioxide and consumer goodness of products made therewith is best created and maintained by providing a buffer of the phosphate type which allows gradual release of active chlorine dioxide without depleting the reservoir while at the same time maintaining good organoleptic properties.

Compositions containing stabilized chlorine dioxide for prevention or treatment of oral disease have been described and taught by Ratcliff (US 4689215, US 4696811, US 4786492, US 4788053, US 4792442, US 4793989, US 4818519, US 4851213, US 4855135, US 4886657, US 4889714, US 4925656). Compositions containing stabilized chlorine dioxide and phosphate for prevention or treatment of oral disease have been described and taught by Ratcliff in US 5200171, US 5348734 and for the treatment of abnormal conditions of the epithelium of bodily orifices in US 5489435, US 5618550, US 5811115, US 5834003, US 5902575, US 5935592, US 6017554. For reference, an unflavored 0.100% (weight/volume) stabilized chlorine dioxide oral rinse, buffered by trisodium phosphate at pH 6.4 to 7.0 as indicated by Ratcliff, contains approximately 1-20 ppm (parts per million) of chlorine dioxide prior to activation (Silwood 2001, Grootveld 2001). Ratcliff prior art patents do not however indicate how to achieve or to maximize chemical stability of stabilized chlorine dioxide when a flavoring agent is added to the composition and none of these prior art patents reference the roles of glycerin or polysorbate in stability of stabilized chlorine dioxide or chlorine dioxide. Ratcliff prior art does not specifically mention: 1) using polysorbate as an emulsifier for the flavoring agent, 2) excluding glycerin or ethoxylated hydrogenated castor oil as a surfactant in the composition, and 3) the specific pH range of the present invention (Ratcliff prior art specifies pH of 6.0 to 7.4; the present invention specifies a pH range of pH 6.5 to 8.0).

Efforts to add flavoring agents to Ratcliff compositions have resulted in predominantly two alternate outcomes: Outcome 1) SCD compositions where the stabilized chlorine dioxide is not stable and chlorine dioxide gas is readily formed, or Outcome 2) SCD compositions which have visible aqueous and oil phases that are not miscible and heterogeneous SCD compositions are formed. Outcome 1 is believed to occur due to the oxidizing natures of chlorine dioxide ($ClO_2$) and the chlorite ion ($ClO_2^-$) which results in the degradation of the flavoring agent or the flavoring agent causing

$ClO_2$ and $ClO_2^-$ to become unstable. Outcome 2 is thought to happen as a result of the hydrophilic and hydrophobic characteristics of aqueous stabilized chlorine dioxide and flavor oil which renders each phase insoluble in the other phase. An example of this is an oral rinse (pH 6.4 to 7.0), a 0.100% (w/v) stabilized chlorine dioxide oral rinse buffered by trisodium phosphate, which is translucent when flavored. Even when a polysorbate sold under the trademark Tween™ is used in oral care compositions containing stabilized chlorine dioxide, this does not assure clarity of the composition. For example, in the case of a 0.100% (w/v) stabilized chlorine dioxide oral spray (pH 7.3 to 7.8 and buffered with sodium citrate, containing glycerin, polysorbate-20, and mint flavor oil) the resulting composition appears translucent (not clear and water-like).

Witt, et al, instructs methods and oral care compositions to prevent or treat "diseases of the oral cavity (e.g. plaque, gingivitis, periodontal disease), breath malodor, and for whitening teeth, in humans or other animals, a safe and effective amount of chlorite ion" (US 6077502, US 6132702, US 6235269, US 6251372, US 6264924, US 6350438). In addition to explicitly stating that the chlorite ion is the compound of interest to induce therapeutic and cosmetic benefits in the oral cavity, the Witt et al. patents specifically limit the invention to compositions that are essentially free of chlorine dioxide that is defined as less than 50 ppm of chlorine dioxide. To further illustrate this point, the preferred pH of compositions listed in these patents is greater than 7.5 and even more preferably greater than 8, where the chlorite ion is known to be more stable and chlorine dioxide gas is not readily formed. In addition, US 6696047 states that US 6077502, US 6132702, and US 6251372 are intended to teach "compositions for the delivery of chlorite ion to the oral cavity for efficacy, wherein the compositions are specifically formulated to avoid or minimize the production of chlorine dioxide or chlorous acid." Witt et al. mentions the use of humectants, including glycerin, in the oral care compositions as well as flavoring agents (like oil of peppermint), and nonionic surfactants (like that sold under the trademark Tween™). Witt et al. generally mentions buffers for dual phase compositions, but buffers are not essential components of the aforementioned Witt et al. inventions.

US 6582682 teaches how to flavor oral care compositions containing stabilized chlorine dioxide. Unlike the present invention, US 6582682 specifically instructs the use of glycerin and ethoxylated hydrogenated castor oils (at pH 6.3 or above, with claims for compositions of pH 6.5 to 10), as key components that allow for use of flavoring agents "which are stable and are not affected by oxidation reactions or exhibit chlorine odor and yellowing discoloration." Per previous statements about the stability of the chlorite ion at basic pH, it is expected that at basic pH (pH greater than 8.0) the flavoring agent would be stable in aqueous sodium chlorite and chlorine dioxide would not be readily formed. However, at the more neutral pH claimed in US 6582682 and within the range of the present invention, it will be shown that by excluding glycerin and ethoxylated hydrogenated castor oil, the stability of flavored oral care compositions containing a chlorine dioxide source improves over what is claimed in US 6582682. This exclusion is not obvious from the prior art.

Scott, et al, (US 6696047) teaches stable oral care compositions containing the chlorite ion essentially free of chlorine dioxide and chlorous acid. Scott indicates that "essentially free of chlorous acid or chlorine dioxide" is used to mean "a composition which comprises very low levels, e.g. less than about 2 ppm, preferably less than 1 ppm of chlorine dioxide or chlorous acid, using known analytical methods for measuring chlorine dioxide or chlorous acid including highly specific electron spin resonance (ESR) spectroscopy." Further, US 6696047, like Witt et al., indicates that a "safe and effective amount" of chlorite ion, not chlorine dioxide, is the "essential ingredient" where "safe and effective" means "an amount of chlorite ion, high enough to significantly (positively) modify the condition to be treated, but low enough to avoid serious side effects..." According to the patent, the invention is designed to be contained in two phases, which may be combined in a single phase from about 1 minute to 1 hour before use or during use of the composition. It should be noted that in US 6696047 a nature of speculation exists to include compositions with a pH higher than 7, since all of the stability data and respective embodiments presented have a pH of 8 or higher. The results of all examples presented in Scott exhibit an increase of the chlorite ion over time, which indicates the conversion of chlorine dioxide to chlorite, at the expense of chlorine dioxide gas, which is in opposition to the spirit of the present invention. It should be noted, that unlike the previously discussed teachings of Witt and Scott, the present invention, in the form of an oral rinse [at a concentration of 0.100% (w/v)], is believed to release approximately 35 to 100 ppm chlorine dioxide gas upon use of the composition in the oral cavity. Release of chlorine dioxide gas (not the chlorite ion) is believed to induce the mechanism of action of the present invention; and release of chlorine dioxide gas is expected to occur with use in the oral cavity of other embodiments (such as an oral spray, toothpaste, or gel) of the invention.

Oxyfresh Worldwide, Inc., Idaho, USA, markets and distributes flavored oral care products containing stabilized chlorine dioxide or sodium chlorite. Oxyfresh toothpaste (including Oxyfresh Fluoride Toothpaste, Oxyfresh Non-Fluoride Toothpaste, Oxyfresh Power Paste, and Oxykids Bubble Gum Flavored Toothpaste) all contain either glycerin or hydrogenated castor oil. Further, one of these toothpaste products (Oxykids Bubble Gum Flavored Toothpaste) contains hydrogenated castor oil. Oxyfresh also produces flavored oral rinse products (including Oxyfresh Fresh Mint Mouthrinse, Oxyfresh Fluoride Mouthrinse with Fresh Mint, Oxyfresh Patented Zinc Mouthrinse with Fresh Mint, and Oxyfresh Power Rinse with Lemon Mint Flavor). Oxyfresh Power Rinse with Lemon Mint Flavor also contains hydrogenated castor oil. Given the known ingredients of these products and the known interaction of their various components, one skilled in the art would not expect the Oxyfresh products to maintain stability of the stabilized chlorine dioxide ingredient for a reasonable period of time as defined by the criteria for the present invention. In the case of the toothpaste, the use of glycerin or hydrogenated castor oil may cause the stabilized chlorine dioxide to be unstable and degrade over time. Xylitol is used in all the oral rinse products, and research has shown xylitol to degrade stabilized chlorine dioxide (evidence to support this conclusion will be provided below). Finally, two products (Oxyfresh Power Rinse with Lemon Mint Flavor and Oxykids Bubble Gum Flavored Toothpaste), use polyethylene glycol as a humectant; the nonionic, non-polar nature of polyethylene glycol is believed to affect the stability of stabilized chlorine dioxide adversely, further destabilizing these compositions. In sum, a careful analysis of the foregoing products indicates that the formulations contained therein do not conform to the specifications of the present invention and composition and thus do not offer the balance between a sodium chlorite reservoir and free chlorine dioxide gas that is believed to be critical to flavor stability.

TheraBreath, Los Angeles, CA, also has several flavored toothpastes, containing stabilized chlorine dioxide, however these products also contain glycerin (and xylitol), believed to result in chemically unstable oral care formulations that exhibit rapid degradation of stabilized chlorine dioxide. In the case of TheraBreath Fluoride toothpaste, the pH is alkaline (pH 8.60) which indicates chlorite will not readily form chlorine dioxide, violating the intent of this present invention. Analysis of the Therabreath products, like those of Oxyfresh, indicates that the formulations contained therein do not conform to the specifications of the present invention and composition.

Finally, White Pine Medical, Inc., Park City, Utah and Bio-Cide International, Inc., Norman, OK, market a flavored mouthwash containing 0.16% w/w sodium chlorite (with 0.10% w/w chlorine dioxide efficacy), glycerin, mint oil, and 1.13% w/w citric acid at pH 5.65. (Shinada 2010) With the inclusion of glycerin and an acidic pH, this mouthwash is outside the spirit of the present invention and would not allow for long-term stability of the chlorine dioxide source as defined in this specification.

Compositions containing stabilized chlorine dioxide have been proposed for ophthalmic use, in particular in combination with therapeutics or to disinfect contact lenses. In US 2005/0196370, Yu teaches stable ophthalmic oil-in-water emulsions with sodium hyaluronate for alleviating dry eye. US 2005/0196370 specifically teaches: 1) compositions where the surfactant (such as a polyethylene glycol surfactant or Polysorbate 80) emulsifies the polar oil component in a manner that allows for little to no mechanical homogenization of the oil phase in the aqueous phase and 2) a method to prepare these self-emulsifying compositions to alleviate dry eye. [It should be noted that the inventors define "mechanical homogenization" as occurring at shear speeds greater than 1000 rpm]. These self emulsifying compositions also allow for the inclusion of stabilized chlorine dioxide (SCD) in the composition as a source of chlorite, to act as a preservative and maintain sterility of the composition - rather than to incorporate SCD as a source of chlorine dioxide to facilitate antimicrobial kill at the site of drug delivery. Ophthalmic applications do not involve the use of excipients in the adjustment of taste. Therefore, the function of these excipients, as well as the concentrations used and manner in which these excipients are incorporated into the composition, is fundamentally different compared with present invention.

US 2008/0269353 teaches a composition to prevent the generation of chlorine dioxide in a liquid preparation for ophthalmic use containing a chlorite (pH 3 to 9), where chlorite acts as a preservative to maintain sterility of the composition. The composition specifically comprises chlorite ion, a stabilizer for the chlorite ion, and a liquid preparation for ophthalmic use comprising the composition (where the mechanism of action results from therapeutic compound(s) in the liquid preparation). The source of chlorite ion in the invention may be a salt of chlorous acid (including sodium chlorite), and the preferred concentration of chlorite is 0.0001% to 0.1% (w/v). The stabilizer incorporated into the composition may include one or more of the following: creatinine, geraniol, glucose, tocopherol acetate, oxyquinoline sulfate, sugar alcohol, or polyoxyethylene sorbitan fatty acid esters. It is clearly stated in the disclosure of the invention, that these stabilizers maintain chlorite to prevent the formation of chlorine dioxide because chlorine dioxide is irritating to the eyes - Table 1 of US 2008/0269353 illustrates these compositions generate 0.02 ppm or less chlorine dioxide (of which one composition contains polysorbate 80) thus substantiating the purpose of the invention. As with the preceding patent, US 2008/0269353 does not involve the use of excipients in the adjustment of taste, which is of no significance in ophthalmic preparations. Therefore, the function of these excipients, as well as the concentrations used and manner in which these excipients are incorporated into the composition, is fundamentally different compared with present invention.

US 2003/129144 discloses stable topical oral compositions comprising at least a minimally effective amount of chlorite ion. The pH of the final composition is greater than 7, the composition is essentially free of chlorine dioxide, and the composition is stable against loss of chlorite for a period of at least one year under normal storage conditions at about 25 DEG C. or for a period of 3 months under accelerated storage conditions at about 40 DEG C. The compositions may comprise a flavor system which is stable against degradation by chlorite. EP0613678 describes a stable mouth wash or dentifrice composition containing stabilized chlorine dioxide and phosphates for reducing the motility of and killing microbial pathogens. The preferred concentration ranges are between about 0.005% - 0.5% chlorine dioxide, and between about 0.02% - 3.0% phosphate.

US2010221198 discloses a composition of stabilized chlorine dioxide at a concentration range of about 0.005% to about 0.800% (w/v) at a pH in the range of 6.0 to 7.4 for the prevention of oral diseases caused by dental biofilm and plaque accumulation, such as gingivitis and periodontitis, through bactericidal properties.

SUMMARY OF THE INVENTION

The present invention teaches a composition and method as set out in the claims to flavor oral care compositions containing a chlorine dioxide source, where, over a reasonable period of time, the composition retains: 1) chemical stability of the chlorine dioxide source and pH and 2) the consumer goodness qualities of taste, appearance, and color. The composition may be in the form of a rinse, paste, gel, spray, varnish, powder, impregnated floss, dissolving tablet or similar.

The composition includes:

1. Chlorine dioxide source (including but not limited to sodium chlorite, chlorite ion, chlorine dioxide, stabilized chlorine dioxide (SCD) or similar)
2. Flavoring System containing:

a. Polyoxyethylene sorbitan ester (including but not limited to the product sold under the trademark Tween (polysorbates) or similar)
b. Flavoring agent (including but not limited to oil of peppermint, oil of spearmint, oil of wintergreen, oil of cinnamon, eucalyptus oil or similar)

3. Phosphate buffer (including but not limited to sodium phosphate tribasic, sodium phosphate dibasic, sodium phosphate monobasic, tetrasodium pyrophosphate, or mixtures thereof)
4. Sweetener (including artificial sweeteners, such as sucralose, sodium saccharin, or similar or natural sweeteners, such as sucrose, xylitol, or similar, or mixtures thereof)
5. Water

In the flavoring system the flavoring agent is emulsified in the polyoxyethylene sorbitan ester.

The oral care composition should be essentially free of glycerin (also known as glycerol, or 1,2,3 propanetriol). The oral care composition should be essentially free of castor oil and castor oil derivatives (including hydrogenated castor oils and ethoxylated hydrogenated castor oils). The pH of the oral care composition should be equal to or higher than pH 6.5 and equal to or lower than pH 8.0; with a preferred pH of pH 6.5 to 7.5, and an even more preferred pH of pH 7.3 to 7.5. A weak organic acid (such as acetic acid, fumaric acid, citric acid, or similar) may be added to the composition to adjust the final pH of the composition. The oral care composition may contain a fluoride ion source including but not limited to sodium fluoride, sodium monofluorophosphate, stannous fluoride or similar fluoride ion source.

The method instructs the emulsification of a flavoring agent (such as peppermint oil) in a polyoxyethylene sorbitan ester (such as the product sold under the trademark Tween) to form a flavoring system, which when added to aqueous, phosphate-buffered compositions containing a chlorine dioxide source (at pH 6.5 to 8.0, essentially free of glycerin, and essentially free of castor oil and castor oil derivatives) form flavored oral compositions that retain chemical stability and consumer goodness over a reasonable period of time.

The composition may be used to prevent or treat diseases and/or conditions of the oral cavity, such as dental caries, periodontal disease, osteonecrosis of the jaws, oral candidiasis and mucositis, and other bacterial, fungal, or inflammatory diseases and conditions of the oral cavity known to those in the art. The composition may be used to eliminate oral malodor and cleanse the oral cavity. The composition may be used in humans or pets.

In sum, this invention teaches a composition and method to maintain the equilibrium between a chlorite reservoir and free chlorine dioxide, which allows the gradual release of active chlorine dioxide, without depleting the reservoir while at the same time maintaining good organoleptic properties of the composition.

A primary object of the present invention is to provide an oral care composition having chemical stability and an acceptable consumer quality of taste, appearance and color for a reasonable period of time.

Another object of the present invention is to provide an oral rinse having clarity throughout storage for a reasonable period of time.

Still another object of the present invention is to provide an oral rinse having a consumer acceptable odor for a reasonable period of time.

Yet another object of the present invention is to maintain a chemical equilibrium between the chlorite and the chlorine dioxide in the stabilized chlorine dioxide of an oral care composition to ensure generation of chlorine dioxide gas upon use in the oral cavity.

Still another object of the present invention is to provide an oral care composition for releasing chlorine dioxide gas upon use in the oral cavity and having a phosphate buffer to achieve and maintain a pH in the range of about 6.5 to about 8.0 for a reasonable period of time.

A further object of the present invention is to provide a stabilized chlorine dioxide oral care composition including a non-ionic, polar surfactant such as polyoxyethylene sorbitan ester to maintain the balance between chlorite and chlorine dioxide present.

A still further object of the present invention is to provide residual organoleptic benefits to a user of stabilized chlorine dioxide based oral care product by including a flavor oil.

A yet further object of the present invention is to provide a natural or an artificial sweetener to a stabilized chlorine dioxide oral care composition to retain the original taste of the composition for a reasonable period of time.

These and other objects of the present invention will become apparent to those skilled in the art as the description thereof proceeds.

## DESCRIPTION OF THE PREFERRED EMBODIMENT

Consumers expect oral care products, such as toothpastes, oral rinses, dentifrice gels, and oral sprays, to be chemically stable (for active and/or cosmetic ingredients) and to have acceptable and/or agreeable consumer goodness qualities (such as taste, odor, appearance, or color) for a reasonable period of time, usually from the time of manufacture, through the time of transport to the retail store, through the time on the shelf of the retail store, and throughout the normal period of usage by the consumer. If consumer goodness qualities degrade or decline during this period of time, the consumer may regard the product as spoiled or no longer fresh and/or the chemical stability of the product may be compromised.

Oral care products containing a chlorine dioxide source may be perceived by the consumer as having a disagreeable taste if a flavoring agent is not added to the composition. If an oral care product contains both a chlorine dioxide source and a fluoride ion source, the product taste may be regarded as even more objectionable due to the presence of fluoride. The primary reason for the disagreeable taste encountered in these products is that chlorine dioxide is a powerful oxidizing agent which is known to oxidize, alter or eliminate many flavoring agents. During this process, the chlorine dioxide source may also be degraded which compromises the chemical stability of the product.

A current practice to address this flavoring problem is to provide the consumer with a separate vial containing flavor, which may be added to the unflavored oral care product immediately prior to use of the product; thus minimizing the time that the flavoring agent interacts with the chlorine dioxide source. It is however desirable for oral care products, containing a chlorine dioxide source, to be both permanently flavored and chemically stable for a reasonable period of time, such as from the time of product manufacture through long-term usage of the product by the consumer. It is desirable because an acceptable taste and chemically stable product increases the marketability of these products to consumers and/or may reduce the cost of manufacturing these products by eliminating the need for separate vials/containers for the flavoring agent and the unflavored product.

**Chlorine Dioxide Source**

Properties of Chlorine Dioxide and the Chlorite Ion

Chlorine dioxide ($ClO_2$) is an uncharged molecule with a three-electron bond, which is a potent oxidizer of various classes of compounds including aldehydes, thiols, and phenols. The oxidation reaction of compounds by chlorine dioxide may be generalized by the following reaction (Masschelein 1979):

$$ClO_2 + e^- \; -> \; ClO_2^- \; -> \; (+ 4\,e^- + 4\,H^+) \; -> \; 2\,H_2O + Cl^- \quad \text{(Equation 1)}$$

In the above reaction, chlorine dioxide ($ClO_2$) receives an electron from the reductant ($e^-$) and $ClO_2$ is subsequently converted to the negatively charged chlorite ion ($ClO_2^-$). The chlorine atom in chlorine dioxide and the chlorite ion exist in different oxidation states (+4 in chlorine dioxide and +3 in the chlorite ion, respectively). This strong propensity to attract an electron in the oxidation process has led some authorities to classify chlorine dioxide as a free radical, though most of its chemistry is consistent with its classification as a stable though highly reactive molecule.

One characteristic resulting from this difference in oxidative state renders the chlorite ion a "less significant oxidant" compared with chlorine dioxide (Masschelein 1992). Another difference noted by Masschelein is that chlorine dioxide has "strongly bactericidal" properties while chlorite tends to be "bacteriostatic and slightly bactericidal" in nature (Masschelein 1979). Further to this point, it is believed that chlorine dioxide is a superior water disinfectant due to its ability to dissolve as a gas in water and readily diffuse through "hydrophobic lipid bilayers" to interact with "vital centres of organisms" (Masschelein 1992). As a result of its superior properties as an oxidant and disinfectant, chlorine dioxide has been used in a wide variety of applications including: water purification, disinfection of dental unit waterlines, and the prevention and treatment of oral disease and oral malodor. (Masschelein 1979, Lynch 1997, EPA 1999, Masschelein 1992)

To the last point, oral care products containing chlorine dioxide sources, composed of chlorine dioxide and the chlorite ion, have also been used in oral care products to prevent or treat diseases and/or conditions of the oral cavity. These products have been used for their antibacterial properties since bacteria in dental plaque biofilms are the major cause of several oral diseases including gingivitis, chronic and aggressive periodontitis, and necrotizing periodontal diseases. Most oral infections are surface lesions caused by normal residents of the oral microbiota and/or exogenous pathogens that colonize the oral cavity. These infections include but are not limited to dental caries, gingivitis, periodontitis, endodontic lesions, and systemic infections that can have oral manifestations such as tuberculosis and syphilis. In the case of oral malodor, chlorine dioxide and the chlorite ion are oxidizers of volatile sulfur compounds; volatile sulfur compounds (such as hydrogen sulfide, methyl mercaptan, and dimethyl sulfide) are the "principal malodorants" of the oral cavity (Frascella 1998).

Since uncharged chlorine dioxide has more potent oxidative and bactericidal properties compared to the chlorite ion, when a composition containing a chlorine dioxide source is used as an oxidizer or an antibacterial agent in the oral cavity, it is believed that the mechanism of action is primarily induced by chlorine dioxide ($ClO_2$) gas rather than the chlorite ion ($ClO_2^-$). As a result, oral care compositions containing a chlorine dioxide source have mainly been used as a source of chlorine dioxide for the treatment or prevention of oral diseases, such as gingivitis and periodontitis, and to eliminate or reduce precursors of oral disease, like plaque, biofilms, and disease causing microbes. In addition, the more powerful chlorine dioxide has been used to oxidize volatile sulfur compounds to reduce or eliminate oral malodor. In some cases, however, the chlorite ion has been exploited for its lesser, but present, oxidative and antibacterial properties to prevent and/or treat conditions/diseases of the oral cavity.

Due to the stated superior oxidative and antibacterial properties of chlorine dioxide compared to the chlorite ion, for the purpose of the present invention, chlorine dioxide, rather than the chlorite ion, is the preferred chlorine-containing species for preventing and/or treating diseases and conditions of the oral cavity.

## Generation of Chlorine Dioxide from a Chlorine Dioxide Source

Chlorine dioxide ($ClO_2$) exists as a volatile gas when produced and is therefore not ideal for storage. As previously stated, however, $ClO_2$ is water soluble and may be dissolved as a gas in water. Therefore, those in the art have stored $ClO_2$ by dissolving chlorine dioxide gas in aqueous solutions. $ClO_2$ gas is then liberated through acidification of the solution. One specific example of such a method involves the use of aqueous stabilized chlorine dioxide (SCD) solutions which contain the chlorite ion ($ClO_2^-$), in the form of sodium chlorite ($NaClO_2$), as the predominant chlorine species. SCD solutions may contain other chlorine species such as the chloride ion ($Cl^-$), chlorate ion ($ClO_3^-$), and chlorine dioxide ($ClO_2$) in addition to the chlorite ion ($ClO_2^-$). (Masschelein 1979, EPA 1999, Grootveld 2001)

The acidification of chlorites to produce chlorine dioxide may be generalized by the following reaction (Masschelein 1979):

$$5\,ClO_2^- + 4\,H^+ \;=\; 4\,ClO_2 + 2\,H_2O + Cl^- \;\text{(Equation 2)}$$

Chlorine dioxide is generated from chlorite ion in this process as are the chloride ion and water, with minor and variable amounts of chlorate ion also present in solution. The amount of uncharged chlorine dioxide released from SCD solutions depends importantly on the initial concentration of chlorite ion in solution and also on the pH and maintenance of pH, as by buffers, of the solution (as expanded on in the following paragraph regarding buffers). The yield of $ClO_2$ gas is also dependent on, but not limited to, the following parameters: 1) the concentration of the acid that is applied to the SCD solution for acidification and 2) the strength of the acid that is applied to the SCD solution for acidification (e.g. sulfuric acid versus acetic acid). (Masschelein 1979)

Based on Equations 1 and 2 above, it may also be inferred that when the concentration of the chloride ion ($Cl^-$) is greatest in a SCD solution that this chemical state is indicative of: 1) complete consumption of chlorine dioxide as an oxidizer (Equation 1) or 2) the complete generation of chlorine dioxide gas via acidification (Equation 2). As a result, the greatest amount of chlorine dioxide gas released by a SCD solution is expected to be generated when the greatest amount of chloride ion ($Cl^-$) is present in solution. Thus the resulting concentration of chloride ion is an index of loss of the chlorine dioxide gas that is the prime beneficial entity of this invention.

## Phosphate Buffers and Buffering Systems

"A buffer solution is a solution that resists changes in pH when small quantities of an acid or an alkali are added" (Lange 2005). A buffer solution that is acidic has a pH lower than 7; a buffer solution that is alkaline has a pH higher than 7. In aqueous solutions (0.1 molar aqueous solution at 25°C), sodium phosphate buffers have a pH ranging from acidic to alkaline. More specifically, sodium phosphate dibasic ($Na_2HPO_4$) has a pH of 9.1 (1% aqueous solution at 25°C), sodium phosphate monobasic ($NaH_2PO_4$) has a pH of 4.5 (0.1 molar aqueous solution at 25°C), and sodium phosphate tribasic ($Na_3PO_4$) has a pH of 11.9 (1% aqueous solution). (Merck 1996) Masschelein states that "by maintaining the pH at about 5 to 6 by phosphate buffers such as NaH2PO4, $Na_2HPO_4$, $Na_4P_2O_7$, and polyphosphates, the formation of $ClO_2$ may be slowed down" (Masschelein 1979).

Oral care compositions containing stabilized chlorine dioxide can and have been buffered, to near neutral pH, with phosphate compounds composed of the following buffering systems: 1) a weak acid (such as monopotassium phosphate) and a strong base (such as sodium hydroxide), 2) a weak inorganic acid (such as sodium phosphate monobasic) and a slightly alkaline phosphate compound (such as sodium phosphate dibasic), or 3) a weak organic acid (such as citric acid) and an alkaline compound (such as sodium phosphate tribasic). Compositions containing stabilized chlorine dioxide and phosphate for prevention or treatment of oral disease have been described and taught by Ratcliff in US 5200171, US 5348734 and for the treatment of abnormal conditions of the epithelium of bodily orifices in US 5489435, US 5618550, US 5811115, US 5834003, US 5902575, US 5935592, US 6017554.

**Excipients**

In general, excipients are "any more or less inert substance added to a drug to give suitable consistency or form to the drug" (Merck 2006). Excipients of oral care compositions may exist as a gas, liquid, and/or solid; and these compounds may be natural or synthetic. Such excipients include materials like saccharides (mono-, di-, oligo-, poly-, etc.), inorganic compounds, fats, oils and hydrocarbons, amongst others, and synthetic or semi-synthetic derivatives of these materials.

Each excipient: 1) confers specific properties to the composition (such as a moisturizing effect, flavor, or color), 2) may be used in a range of concentrations to achieve these specific properties, and 3) may be incorporated into the composition using different methods dependent on the chemical nature/structure of ingredients in the composition (for example, excipients that are oils may have to be emulsified to solubilize the compound into an aqueous composition).

Each excipient: 1) should not diminish the safety or effectiveness of the active ingredient(s) and 2) should also be compatible with other excipients. The selection of excipient(s), that are chemically compatible with the active ingredient(s) and other excipients, ensures the oral care composition is safe, effective and has physical properties (such as taste and appearance) that are acceptable to the user-making it superior to other similar compositions that may contain an excipient(s) that renders the active ingredient unstable or the composition unacceptable to the user (for example, due to an undesirable taste or appearance).

The focus of the present invention in part considers the structure, function, and compatibility of different types of excipients (such as flavoring agents) and a chlorine dioxide source, and teaches how to select and incorporate excipients to formulate stable, flavored oral care compositions. The following sections provide a general overview and examples of different types of excipients that are critical to or detrimental to creating stable, flavored oral care compositions containing a chlorine dioxide source of the present invention.

**Flavoring System: Emulsifiers/Surfactants**

Surfactants (or surface-active agents) are "agents that modify interfacial tension of water, usually substances that have one lipophilic and one hydrophilic group in the molecule; includes soaps, detergents, emulsifiers, dispersing and wetting agents, and several groups of antiseptics" (MeSH 2011).

Surfactants are commonly-used excipients in oral care compositions to help clean the teeth and to provide a foam that may help to carry away debris. Sodium lauryl sulfate is an anionic surfactant often present in oral care compositions, such as toothpastes, because it is known to have significant antibacterial properties, including the presumption that it can penetrate and dissolve plaque. Another common though less powerful surfactant, the sequestering agent tetrasodium pyrophosphate (TSPP), is known to remove calcium and magnesium from the saliva and immature plaque, so it cannot remain on teeth to form the insoluble deposit called tartar (calcified plaque). TSPP is alkaline and has a bitter taste, requiring additional flavorings to be added to an oral care composition to mask its undesirable flavor. (Sci-Toys 2011)

This invention, however, teaches that a preferred class of surfactants is the nonionic type described below.

Polyoxyethylene sorbitan esters, or polysorbates, are nonionic surfactants (surface-active agents) that are derivatives of sorbitan esters. (For reference, sorbitan esters are partial esters of fatty acids such as lauric, palmitic, or stearic acid.)

Polysorbates include but are not limited to, Polysorbate 20 (Tween 20) and Polysorbate 80 (Tween 80). These surfactants are well known to those in the art as emulsifiers and dispersing agents. (Merck 1996, Merck 2006)

Polyethylene glycols (also known as PEG's), polyoxyethylene alcohols, and polyoxyethylene fatty acid esters are also types of non-ionic surfactants often classified as emulsifiers. Polyethylene glycols are "liquid and solid polymers of the

general formula H(OCH$_2$CH$_2$)$_n$OH, where n is greater than or equal to 4" and include Polyethylene glycol 200, Polyethylene glycol 400, Polyethylene glycol 600, Polyethylene glycol 1500, Polyethylene glycol 4000, and Polyethylene glycol 6000 (Merck 2006). Polyoxyethylene alcohols are polyethylene glycol fatty alcohol ethers and are generally R(OCH2CH2)$_n$OH "where R is a long chain alkyl group or mixture of alkyl groups" and include products sold under the trademarks Alfonic™, Bio Soft™, Brij™, among others (Merck 2006).

Castor oil may be used as a surfactant and is a triglyceride of fatty acids, approximately 78% ricinoleic, 7% oleic, 3% linoleic, 2% palmitic, 1% stearic, and trace amounts of dihydroxystearic acids. (Merck 2006) Castor oil may be hydrogenated to form hydrogenated castor oils. Ethoxylated hydrogenated castor oils, which can also act as types of surfactants, are formed through the reaction of ethylene oxide and hydrogenated castor oil. The ethoxylation number of the surfactant indicates the number of ethoxy groups (C$_2$H$_5$O-) per castor oil molecule. Examples of ethoxylated hydrogenated castor oils include but are not limited to surfactants sold under the trademark, Cremophor® RH (specific examples include, Cremophor RH-40 and Cremophor RH-60).

The purpose of the non-ionic surfactant in the flavoring system of the present invention is to emulsify the flavoring agent and to prevent degradation of the flavoring agent by the chlorine dioxide source.

**Flavoring System: Flavoring Agents**

Flavoring agents are excipients that are "added to foods and medicine to improve the quality of taste" (MeSH 2011). Flavoring agents may be natural, natural and artificial, or artificial. Some of the most common natural flavoring agents, which are derived from plants, include oils such as peppermint oil and spearmint oil. Peppermint oil is a volatile oil and derivative of Mentha piperita. Spearmint oil is also a volatile oil, whose major component is carvone, and is a derivative of menthe spicata. Other examples of flavor oils derived from plants include but are not limited to: oil of cinnamon, oil of wintergreen, and oil of eucalyptus. These flavor oils can be extracted naturally, formulated with natural and artificial components, or synthetically produced.

As used in oral care compositions, flavoring agents impart a flavor profile for the composition such as a peppermint, spearmint or wintergreen taste, depending upon the flavoring agent used.

**Sweeteners**

Sweeteners (or sweetening agents) are "substances that sweeten food, beverages, medications, etc., such as sugar, saccharine or other low-calorie synthetic products" (MeSH 2011). Sweeteners may be natural (generally, monosaccharides, disaccharides, short-chain polysaccharides) or artificial (synthetic). Examples of natural sweeteners include but are not limited to, sucrose, lactose, glucose, maltose, and fructose. Problems may arise with the use of natural sweeteners in compositions intended for human use. These sweeteners may: 1) promote tooth decay (as cariogenic bacteria can metabolize these sugars which may result in tooth demineralization and eventual tooth decay) and 2) cause weight gain (as these sugars are high in calories).

Xylitol, sorbitol, and mannitol are also natural sweeteners but are considered "sugar alcohols." United States (US) Title 21 Part 101.9 (Nutrition Labeling) defines sugar alcohols as "the sum of saccharide derivatives in which a hydroxyl group replaces a ketone or aldehyde group and whose use in the food is listed by FDA (e.g., mannitol or xylitol) or is generally recognized as safe (e.g., sorbitol)." Artificial sweeteners have been developed to address the dietary and health problems associated with natural sweeteners. Such artificial sweeteners include but are not limited to sodium saccharin and sucralose.

As used in oral care compositions, sweeteners help to enhance the flavor profile of the composition and prevent it from being bitter and unpalatable to the user.

**Glycerin**

Glycerin (C$_3$H$_8$O$_3$), also known as 1,2,3 Propanetriol or glycerol, contains three hydroxyl groups (-OH) and is "obtained from oils and fats as byproduct in the manuf(acture) of soaps and fatty acids" (Merck 1996). Glycerin is a widely used excipient in the cosmetic and pharmaceutical industries for its various chemical and physical properties. One such property is that glycerin is able to absorb moisture from the ambient environment. Glycerin is also miscible in both alcohol and water. Glycerin has a "sweet warm taste" and is "about 0.6 times as sweet as cane sugar"; therefore glycerin may add sweetness to compositions (Merck 1996). As a result, glycerin is routinely used as a humectant in oral care compositions, such as oral rinses or toothpastes.

**Chemical Stability and Consumer Goodness of the Present Invention**

The present invention teaches a composition and method to flavor oral care compositions containing a chlorine dioxide source, where, over a reasonable period of time, the composition retains: 1) chemical stability of the chlorine dioxide source and pH and 2) the consumer goodness qualities of taste, appearance, and color.

Chemical stability refers to stability of: 1) the chlorine dioxide source and 2) pH. Chemical stability of the composition is achieved if, throughout storage during a reasonable period of time, the composition maintains both:

Criterion 1 (Chemical Stability): 10% or less degradation of the chlorine dioxide source from the time zero value
The inventors believe a chemical equilibrium exists between the chlorine dioxide source and any chlorine dioxide present in the composition, prior to use, and this equilibrium allows for the generation of chlorine dioxide gas upon use of the composition in the oral cavity. The inventors believe that maintaining the chlorine dioxide source at this specified threshold, ensures that the chemical equilibrium between the chlorine dioxide source and chlorine dioxide is maintained enough so that a sufficient amount of chlorine dioxide is generated upon use of the composition in the oral cavity.
and

Criterion 2 (pH): A pH equal to or higher than pH 6.5 and equal to or less than pH 8.0.
Consumer goodness refers to the qualities of: 1) taste, 2) odor, 3) appearance, 4) clarity (if applicable), and 5) color. Consumer goodness of the composition is achieved if, throughout storage during a reasonable period of time, the composition:

Criterion 3 (Taste and Odor): Is essentially free from an undesirable or unpleasant taste and odor (including but not limited to a strong salty taste or bleach like taste or odor) and retains the intended flavor notes and odor of the composition, as known to a person skilled in the art. The presence of an undesirable taste or odor (such as a strong salty taste or bleach like odor) may indicate the generation of chlorine dioxide from the chlorite reservoir.

Criterion 4 (Appearance or Clarity): With reference to appearance, maintains the physical shape intended for the composition, as known to a person skilled in the art (for example, the intended appearance of a toothpaste is a homogeneous semi-solid paste). It is also an objective of the present invention for the consumer goodness of a liquid embodiment, such as an oral rinse, to retain clarity (clear, water-like appearance); however clarity should not limit the presence of a color in the composition if a color is intended.

Criterion 5 (Color): Maintains the intended color of the composition, as known to a person in the art. It is known that the appearance of a yellow color over time indicates the production of enough chlorine dioxide gas to signal the disruption of chemical equilibrium between the chlorine dioxide source and chlorine dioxide. Therefore, unless intended by the formulator, a yellow color in the composition is not desirable to indicate consumer goodness.

A person skilled in the art would know that a reasonable period of time refers to the time (including days, months, or years) a composition is expected to maintain chemical stability and consumer goodness to induce delivery of its intended therapeutic indications and/or cosmetic attributes. For example, in one embodiment, the composition should maintain chemical stability and consumer goodness from manufacture of the composition through twelve (12) months storage under ambient conditions. Ambient conditions are defined as room temperature (20-35°C) and average temperate-zone relative humidity (50%-70%). In another embodiment, storage of the composition under accelerated conditions (typically 40°C and 75% relative humidity) can project real time suitability of a composition for consumer use. For example, if the composition maintains chemical stability and consumer goodness through three (3) months storage under accelerated conditions, this situation indicates that the composition is suitable for consumer use for one (1) year in real time while stored under ambient conditions.

For the purpose of the present invention, a reasonable period of time should be: 1) storage of the composition under ambient conditions for at least six (6) months and/or 2) storage of the composition under accelerated conditions for at least one (1) month.

**Components of the Invention**

A chlorine dioxide source is essential to the present invention, as the inventors believe chlorine dioxide gas, rather than the chlorite ion, is the ingredient which induces any therapeutic or cosmetic mechanism of action of the composition. A chlorine dioxide source, known to those in the art, may include but is not limited to stabilized chlorine dioxide (liquid), sodium chlorite (powder), or the chlorite ion. The preferred chlorine dioxide source for the present invention is stabilized chlorine dioxide. Examples of stabilized chlorine dioxide are Anthium Dioxcide® sold by International Dioxcide or a 5% Stabilized Chlorine Dioxide liquid sold by Bio-Cide International. The main chlorine species in stabilized chlorine dioxide is the chlorite ion ($ClO_2$), but variable and lesser amounts of chlorine dioxide ($ClO_2$), chlorate

(ClO$_3$$^-$), and chloride (Cl$^-$) are also present. The inventors believe a chemical equilibrium exists between the chlorite ion and chlorine dioxide (in the headspace of the container) in stabilized chlorine dioxide; and that this equilibrium should be maintained to ensure generation of chlorine dioxide gas upon use of the composition in the oral cavity.

The inventors believe the amount of chlorine dioxide released from the composition is primarily dependent on the initial concentration of the chlorite ion in the composition and the maintenance of pH, with secondary factors including the initial pH of the composition and the presence of compounds which can be oxidized by the chlorite ion and/or chlorine dioxide (such as alcohols, ketones, and phenols).

A sufficient amount of chlorine dioxide gas must be produced by the composition to induce the therapeutic or cosmetic mechanism of action, upon use of the composition in the oral cavity. The therapeutic mechanism of action (preventing or treating conditions and diseases of the oral cavity) is induced by the antibacterial properties of chlorine dioxide. The cosmetic mechanism of action (minimizing or eliminating oral malodor) is induced by the oxidative properties of chlorine dioxide relative to volatile sulfur compounds as well as by the effect of the flavoring substances themselves.

To produce a sufficient amount of chlorine dioxide gas to induce the therapeutic and/or cosmetic mechanism of action of the composition:

The concentration of the chlorine dioxide source in the final composition should be: 0.050% to 0.600% weight/weight (w/w). The preferred concentration of the chlorine dioxide source in the final composition is 0.100% to 0.500% w/w.

The concentration of the chlorite ion in the final composition should be: 0.050% to 0.600% weight/weight (w/w). The preferred concentration of the chlorite ion in the final composition is 0.100% to 0.500% w/w.

A phosphate buffer is preferred for the present invention to achieve and maintain a pH range of equal to or higher than pH 6.5 or equal to or lower than pH 8.0. A phosphate buffer may include sodium phosphate tribasic, sodium phosphate dibasic, sodium phosphate monobasic, or mixtures thereof. Pyrophosphate salts, such as tetrasodium pyrophosphate (Na$_4$P$_2$O$_7$), which are anti-calculus agents, may also be used as phosphate buffers.

Unlike other buffers, a phosphate buffer is considered preferred for the invention because it is known to the inventors to reliably maintain the specified pH range when the present invention is stored under accelerated or ambient conditions over a reasonable period of time. The basis for this belief is that a phosphate buffer, or mixtures thereof, tend toward an equilibrium (of pH) in solution, which helps maintain the pH of the composition and thereby maintains the equilibrium of the chlorite ion reservoir and chlorine dioxide over long periods of time. As a result, this allows for gradual release of the chlorine dioxide upon use of the composition, without depleting the reservoir, while at the same time maintaining good organoleptic properties of the composition.

Weak acids (like lactic acid) alone are not good buffers and will not maintain the pH specified by the present invention; and thereby will not maintain the equilibrium between the chlorite reservoir and chlorine dioxide. Furthermore, in dual phase compositions where weak acids and the chlorine dioxide source are kept separate, a weak acid may be added to activate the chlorine dioxide source to generate chlorine dioxide gas but it is believed that this reaction occurs too quickly (30 seconds to 1 minute) and that the pH of the composition will rapidly increase and chlorine dioxide gas will cease to be generated in amounts sufficient for therapeutic and/or cosmetic benefit to the user.

The pH range specified by the present invention is based on the known effect of pH on chlorine dioxide, stabilized chlorine dioxide, and the chlorite ion. The pH is known to affect: 1) the maintenance of the chemical equilibrium between the chlorite ion and chlorine dioxide and 2) the generation of chlorine dioxide gas upon use of the composition in the oral cavity. According to EPA (1999), chlorine dioxide "disproportionates under highly alkaline conditions (pH > 9) to chlorite and chlorate", which is represented by Reaction 3:

$$2\ ClO_2 + 2OH^- = ClO_2^- + ClO_3^- + H_2O \qquad \text{(Reaction 3)}$$

In addition, the inventors believe at greater than pH 8.0, the present invention would not generate a sufficient or effective amount of chlorine dioxide gas, from the chlorine dioxide source, to induce a therapeutic or cosmetic mechanism of action in the oral cavity. The inventors believe the chlorite ion would be too stable, at greater than pH 8.0, and would not readily release chlorine dioxide. Conversely, it is known that at acidic pH, the chlorite ion is rapidly oxidized to chlorine dioxide. The inventors believe production of chlorine dioxide due to this oxidation reaction is relatively minimal between pH 6.5 to 8.0 with the chemical equilibrium between the chlorite ion and chlorine dioxide being maintained in this pH range; and that at pH lower than pH 6.5, the production of chlorine dioxide gas is rapid and chlorine dioxide would be lost from the composition. Therefore to avoid this occurrence, the pH of the composition is pH 6.5 to 8.0, with a preferred pH of 6.5 to 7.5, and the most preferred pH being pH 7.3 to 7.5. Within this pH range, it is well established that the chlorine dioxide source will oxidatively consume the flavoring agents otherwise added to oral care products, and therefore the provision of a flavoring agent that is not oxidatively consumed is an essential characteristic of the present invention.

A flavoring system includes: 1) a polyoxythylene sorbitan ester (including but not limited to a composition sold under the trademark Tweens such as Polysorbate 20 and Polysorbate 80) and 2) a flavor oil (including but not limited to oil of peppermint, oil of spearmint, oil of wintergreen, or mixtures thereof, furthermore the flavor oil may be natural, artificial, or natural and artificial). In the present invention, a polyoxythylene sorbitan ester (also known as polysorbate) is a nonionic surfactant used to emulsify the flavor oil to protect the flavor oil from degradation by the chlorine dioxide source. This flavoring system helps maintain both chemical stability and consumer goodness of the composition over a reasonable period of time.

A surfactant is believed to be essential to the invention because flavor oils tend to contain alcohols, aldehydes or ketones which are readily oxidized by chlorine dioxide and chlorine dioxide sources. The proper surfactant counteracts this tendency. Common anionic and cationic surfactants, however, provide so much foaming in a composition such as this invention teaches that the resulting product is not preferred in use. Further, while the inventors do not wish to be bound by theory, what the inventors refer to as a non-ionic, polar surfactant (such as polyoxyethylene sorbitan ester) is preferred to what the inventors refer to as a non-ionic, nonpolar surfactant (like polyethylene glycol) because a non-ionic, nonpolar surfactant may not stabilize in the way a non-ionic, polar long chain surfactant can because of the latter's ability to maintain the balance between chlorite and chlorine dioxide present in the invention.

The flavor oil is essential because it provides the flavor notes and characteristic taste of the composition (such as a peppermint taste) and may provide residual organoleptic benefits to the user. The polyoxythylene sorbitan ester and flavor oil should be mixed together, prior to addition of the flavoring system to the aqueous phase (comprised of the chlorine dioxide source, phosphate buffer, sweetener, and water) of the invention. A single phase composition is the preferred embodiment of the present invention to flavor the composition and ensure chemical stability and consumer goodness of the composition throughout a reasonable period of time:
The concentration of the polyoxythylene sorbitan ester in the final composition should be: 0.001% to 40.000% w/w and the concentration of the flavor oil in the final composition should be: 0.005% to 10.000% w/w.

A sweetener is essential because this component is a key determinant of the consumer goodness of the invention; it specifically determines the quality of taste. A sweetener may include but is not limited to: 1) artificial sweeteners such as sucralose, sodium saccharin, or similar and/or 2) natural sweeteners, such as sucrose, xylitol, or similar. Without a sweetener, the invention tastes bitter, unpleasant, and unpalatable. The sweetener enhances the notes of the flavor oil, prevents the composition from tasting bitter, and helps retain the original taste of the composition over a reasonable period of time.

The concentration of the sweetener in the final composition should be: 0.005% to 2.000% w/w.

Water is essential to the present invention to maintain chemical stability and consumer goodness of the composition over a reasonable period of time. With regard to chemical stability, water can solubilize chlorine dioxide and the chlorine dioxide source in the composition, which maintains the chemical equilibrium and allows for generation of chlorine dioxide gas upon use of the composition. With regard to consumer goodness, water affects appearance by retaining moisture in the composition to ensure it can take on different embodiments, such as an oral rinse (liquid), a toothpaste (semi-solid), or a dentifrice gel (semi solid).

The amount of water in the final composition is the difference between the amount of the total composition and the amount of the sum of all other components of the composition.

## Optional Components of the Invention

A weak organic acid (including but not limited to acetic acid, fumaric acid, citric acid, or similar) may be added to the composition to adjust the final pH of the composition to a pH of equal to or higher than pH 6.5 or equal to or lower than pH 8.0.

A fluoride ion source may be added to the composition. Examples of a fluoride ion source include sodium fluoride, sodium monofluorophosphate, stannous fluoride, or similar. The concentration of the fluoride ion in the final composition should be 0.025% to 0.160% w/w, with the preferred range being 0.080% to 0.150% w/w.

## Effect of Glycerin on Chemical Stability and Consumer Goodness of Oral Care Compositions Containing a Chlorine Dioxide Source

It is believed that when glycerin is added to aqueous, phosphate-buffered compositions containing a chlorine dioxide source (such as stabilized chlorine dioxide), glycerin absorbs water and changes the aqueous nature of the medium-thereby degrading the chlorine dioxide source and resulting in the premature release of chlorine dioxide gas. In order to evaluate and demonstrate the supposed deleterious effect glycerin has on these types of compositions, several formulations were created and tested for chemical stability and consumer goodness. These stability studies and their respective data are presented and described in the following tables and paragraphs. For the following studies, it should be noted that stabilized chlorine dioxide was assayed using known titration methods or ion chromatography; pH was measured using methods known to those skilled in the art (potentiometric); and taste, appearance, color or odor was evaluated using standardized test methods (unless otherwise specified).

**Table 1. Degradation Study of Stabilized ClO$_2$ in Phosphate Buffered Aqueous Solutions Containing Hydroxyl (-OH) Group Excipient Ingredients**

| Degradation Rates of Stabilized ClO$_2$ | |
|---|---|
| -OH Group Ingredient (1 M) | Degradation Rate of Stabilized ClO$_2$ (mM/Day) |
| Glycerol | 0.17 |
| Xylitol | 0.0882 |
| PEG 1000 | 0.08 |
| PEG 200 | 0.0268 |
| PEG 8000 | 0.0195 |
| Sorbitol | 0.0001 |

The degradation study represented in Table 1 was conducted on aqueous, phosphate-buffered solutions (pH 7.0) containing 1 M of one of the listed -OH group ingredient and 20 mM of stabilized chlorine dioxide (SCD). Each respective solution was stored at 40°C for 21 days. Of the ingredients listed, the degradation rate of SCD was greatest in the phosphate-buffered solution containing glycerol (0.17 mM/Day). Also, there was a notable amount of stabilized chlorine dioxide degradation when xylitol was present in the composition. The degradation rate of SCD was the least in the phosphate-buffered solution containing sorbitol (0.0001 mM/Day).

**Table 2a. Formulation for SCD Phosphate-Buffered Solution 1 (with Glycerin) (Note: Unless otherwise indicated, percentages (%) are expressed in weight/weight)**

| SCD Phosphate-Buffered Solution 1 | | | | |
|---|---|---|---|---|
| Stabilized ClO2 (w/v) | Trisodium Phosphate | Glycerin | pH (adjusted with citric acid) | Water |
| 0.10% | 0.30% | 20.00% | 6.8-7.2 | Balance |

**Table 2b. Results on Inspection for SCD Phosphate-Buffered Solution 1 (with Glycerin)**

| Day 1: Results on Inspection of SCD Phosphate-Buffered Solution 1 | |
|---|---|
| Color | Odor |
| Yellow | Pronounced bleach-like |

In SCD Phosphate-Buffered Solution 1 (formulation detailed in Table 2a), the yellow color and bleach-like odor (reported in Table 2b and observed by the tester in an informal inspection) of the aqueous stabilized chlorine dioxide (SCD) solution indicate that chlorine dioxide gas has likely been produced from the SCD solution. As defined by the invention, the consumer goodness of SCD Phosphate-Buffered Solution 1 is already compromised at Day 1 because the yellow appearance is not intended; SCD Phosphate Buffered Solution 1 was formulated to remain clear, water-like in appearance (no color).

**Table 3. Stability of Toothpastes Containing Stabilized Chlorine Dioxide**

| 90-Day Accelerated Data Comparison of Toothpastes | | | | | | | |
|---|---|---|---|---|---|---|---|
| | *Toothpaste 1* | | | | *Toothpaste 2* | | |
| | Stabilized $ClO_2$ (w/v) | pH | Loss of $ClO_2$ from Day 0 | | Stabilized $ClO_2$ (w/v) | pH | Loss of $ClO_2$ from Day 0 |
| **Day 0** | 0.118% | 7.88 | Not Applicable | | 0.1301% | 6.95 | Not Applicable |
| **15 Days** | 0.093% | 7.93 | 21.19% | | 0.1096% | 6.93 | 15.76% |
| **30 Days** | 0.063% | 7.78 | 46.61% | | 0.0952% | 6.92 | 26.83% |
| **60 Days** | 0.010% | 7.72 | 91.53% | | 0.0646% | 6.90 | 50.35% |
| **90 Days** | 0.000% | 7.74 | 100.00% | | 0.0548% | 7.00 | 57.88% |
| *Toothpaste 1* and *Toothpaste 2* were stored under accelerated conditions, where accelerated conditions are 40°C/75% Relative Humidity. | | | | | | | |
| *Toothpaste 1* contains: Water, Hydrated Silica, Sorbitol, Glycerin, Stabilized Chlorine Dioxide, Cellulose Gum, Trisodium Phosphate, Titanium Dioxide, Flavor, Sodium Saccharin | | | | | | | |
| *Toothpaste* 2 contains: Water, Hydrated Silica, Sorbitol, Stabilized Chlorine Dioxide, Cellulose Gum, Dibasic Sodium Phosphate, Titanium Dioxide, Flavor, Sodium Fluoride, Monobasic Sodium Phosphate, Sucralose | | | | | | | |

Table 3 compares the chemical stability of two toothpastes containing stabilized chlorine dioxide while stored under accelerated conditions (40°C/75% Relative Humidity) for 90 Days. Some of the differences between Toothpaste 1 and Toothpaste 2 are:

Toothpaste 2 contains slightly more stabilized chlorine dioxide (0.1301%) at time zero compared with Toothpaste 1 (0.118%).

Toothpaste 1 is buffered by trisodium phosphate to a more alkaline pH of pH 7.88, while Toothpaste 2 is buffered by sodium phosphate dibasic/sodium phosphate monobasic to a more nearly neutral pH of pH 6.95.

Toothpaste 1 contains glycerin, whereas Toothpaste 2 contains no glycerin.

There was complete degradation (100%) of stabilized chlorine dioxide in Toothpaste 1 through 90 days. Only partial degradation (57.88%) of stabilized chlorine dioxide was observed in Toothpaste 2 through 90 days. Therefore, the chemical stability of stabilized chlorine dioxide is markedly improved in Toothpaste 2 over Toothpaste 1. The lack of glycerin in Toothpaste 2 and a more nearly neutral pH appears to help improve stability of chlorine dioxide compared with glycerin containing, alkaline Toothpaste 1. Despite this improvement, the percent degradation of stabilized chlorine dioxide in Toothpaste 2 does not meet Criterion 1 ($\leq 10\%$) defined by the present invention to indicate chemical stability of the chlorine dioxide source. It should be noted that Toothpaste 2 was not prepared to include a polyoxythylene sorbitan ester to emulsify the flavor. In sum, the selection, concentration, compatibility, and function of the excipients in the respective compositions directly affected chemical stability evaluated over what is considered an estimation of a commercially reasonable period of time (based on accelerated storage).

**Oral Care Compositions containing a Chlorine Dioxide Source, Flavor, Glycerin, and Ethoxylated Hydrogenated Castor Oil**

Flavored oral care solutions containing a chlorine dioxide source, phosphate buffer, glycerin, and ethoxylated hydrogenated castor oil (Cremophor RH-40™) at pH 6.4-6.6 (as taught in US 6582682) exhibited one or more of the following characteristics: translucent appearance, pronounced bleach-like odor, salty or bleach-like taste, degradation of stabilized $ClO_2$, and acidic pH. Tables 4-7 detail these formulations, in the form of a single-phase oral rinse, and the respective stability results.

**Table 4. Flavored SCD Oral Rinse Formulations containing Glycerin and Ethoxylated Hydrogenated Castor Oil**

| (Note: Unless otherwise indicated, percentages (%) are expressed in weight/weight) | | | |
|---|---|---|---|
| Flavored SCD Oral Rinse Formulations Containing Glycerin and Ethoxylated Hydrogenated Castor Oil | | | |
| Ingredient | Oral Rinse 1 | Oral Rinse 2 | Oral Rinse 3 |
| Stabilized $ClO_2$ (w/v) | 0.10% | 0.10% | 0.10% |
| Sodium Fluoride | 0.00% | 0.00% | 0.05% |
| Trisodium Phosphate | 0.20% | 0.20% | 0.20% |
| Glycerin | 20.00% | 20.00% | 20.00% |
| Sorbitol | 10.00% | 10.00% | 10.00% |
| Flavor | 0.15% | 0.15% | 1.50% |
| Ethoxylated Hydrogenated Castor Oil | 0.45% | 0.45% | 4.50% |
| Sucralose | 0.20% | 0.20% | 0.20% |
| pH (adjusted with citric acid) | 6.4-6.6 | 6.4-6.6 | 6.4-6.6 |
| Water | Balance | Balance | Balance |

**Table 5. Day 0: Results for Flavored SCD Formulations of Table 4**

| Day 0 Data | | | | | |
|---|---|---|---|---|---|
| Sample ID | Stabilized $ClO_2$ (w/v) | pH | Appearance/Color | Taste | Odor |
| Oral Rinse 1 | 0.100% | 6.39 | Clear | Not Available (N/A) | N/A |
| Oral Rinse 2 | 0.099% | 6.50 | Clear | N/A | N/A |
| Oral Rinse 3 | 0.102% | 6.56 | Translucent | N/A | N/A |

**Table 6. Day 7: Accelerated (40°C/75%RH) Results for Flavored SCD Formulations of Table 4**

| Day 7: Accelerated Data (40°C/75%RH) | | | | | | |
|---|---|---|---|---|---|---|
| Sample ID | Stabilized $ClO_2$ (w/v) | Change of Stabilized $ClO_2$ from Day 0 Value | pH | Appearance/Color | Taste | Odor |
| Oral Rinse 1 | 0.0877% | 12.30% | 6.28 | Clear | Very slightly weaker | Fresh |
| Oral Rinse 2 | 0.0856% | 13.54% | 6.43 | Clear | Not fresh, sweeter | Slight bleachy |
| Oral Rinse 3 | 0.0108% | 89.41% | 5.18 | Translucent | Very weak, bleachy, salty | Not minty, very bleachy |

**Table 7. Day 14: Accelerated (40°C/75%RH) Results for Flavored SCD Formulations of Table 4**

| Day 14: Accelerated Data (40°C/75%RH) | | | | | |
|---|---|---|---|---|---|
| Sample ID | Stabilized $ClO_2$ (w/v) | pH | Appearance/Color | Taste | Odor |
| Oral Rinse 1 | Not Available (N/A) | 5.15 | Clear, slight straw | Sweeter minty | Good |
| Oral Rinse 2 | N/A | 5.25 | Clear | Salty | Slight bleachy |
| Oral Rinse 3 | N/A | 5.08 | Clear | Weak and bland | Very weak |

Oral Rinse 1, 2, and 3 all show degradation of stabilized $ClO_2$ of greater than 10% after 1 week stored under accelerated conditions - with the greatest degradation occurring in the Oral Rinse 3 containing sodium fluoride (89.41% loss of stabilized $ClO_2$). Furthermore, the pH of all formulations started to drop after 1 week, with Oral Rinse 3 becoming acidic with a pH of 5.18 coinciding with the significant loss of stabilized $ClO_2$. These results further substantiate that acidic pH results in degradation of the chlorine dioxide source. As defined by the present invention, these formulations do not meet the criteria for chemical stability or consumer goodness.

It is not believed the humectant, sorbitol, significantly contributes to instability of stabilized $ClO_2$. This assumption is due to the fact that the degradation rate of $ClO_2$ in the presence of sorbitol is low (Table 1) and also because sorbitol was formulated and tested in a phosphate buffered aqueous stabilized chlorine dioxide formulation similar to Phosphate-Buffered Solution 1 (without glycerin, data not shown) and this formulation remained clear and did not have a pronounced bleach-like odor.

**Exclusion of Castor oil and Castor Oil Derivatives**

Castor oil and castor oil derivatives (including hydrogenated castor oils and ethoxylated hydrogenated castor oils) are excluded as emulsifiers for the present invention because it is believed the structure of these compounds do not make them compatible with chlorine dioxide sources or chlorine dioxide. It is believed that the unsaturated bonds in castor oil, as well as the residual unsaturation in castor oil derivatives, effectively consume a portion of the activity of chlorine dioxide, thus reducing its effectiveness. This belief has been demonstrated, in part, through experimentation with ethoxylated hydrogenated castor oil as an emulsifier in flavored oral care compositions containing a chlorine dioxide source. As demonstrated by stability data for Oral Rinse 1, 2 and 3 the use of ethoxylated hydrogenated castor oil (Cremophor RH-40™) resulted in formulations unstable for stabilized $ClO_2$ and pH; and/or consumer goodness was compromised. When glycerin was excluded as a humectant and ethoxylated hydrogenated castor oil (Cremophor RH-40™) was included in a flavored phosphate-buffered, aqueous stabilized $ClO_2$ composition (data not shown), the resulting composition experienced a pH drop over time and the composition took on a yellow color - indicating the release of chlorine dioxide gas.

**Stability of the Present Invention and Improvement over Prior Art**

According to the previously defined criteria for chemical stability (Criterion 1 and Criterion 2) and consumer goodness (Criterion 3, Criterion 4, and Criterion 5), the flavored oral care compositions taught by the present invention may retain chemical stability and consumer goodness for a period of up to six (6) months while stored under accelerated and/or twelve (12) months while stored under ambient conditions. This assertion is demonstrated in Tables 8-10 below which contain stability data for examples of the present invention, Oral Rinse 4 and Oral Rinse 5. [Note: Accelerated testing serves as a universally recognized proxy for long periods of ambient testing.]

Oral Rinse 4 is a flavored, non-fluoride oral care composition containing the following ingredients: water, stabilized chlorine dioxide (0.100% w/v), trisodium phosphate, peppermint oil and polysorbate, and citric acid. (It should be noted that this formulation contains a very small amount of the humectant, propylene glycol.)

Stability data for Oral Rinse 4, while stored under accelerated and ambient conditions, are presented in Table 8 and Table 9 below.

**Table 8. Six (6) Month Accelerated (40°C/75%RH) Data for Oral Rinse 4**

| Six (6) Months Accelerated Data (40°C/75%RH) for Oral Rinse 4 | | | | | | |
|---|---|---|---|---|---|---|
| Time Point | Stabilized $ClO_2$ (w/v) | Change of Stabilized $ClO_2$ from Day 0 Value | pH | Appearance/Color | Taste | Odor |
| Day 0 | 0.099% | Not Applicable | 7.22 | Colorless clear water-thin liquid | Light minty | Minty |
| 1 month | 0.090% | 9% | 7.27 | Colorless clear water-thin liquid | Light minty | Minty |
| 2 months | 0.090% | 9% | 7.35 | Colorless clear water-thin liquid | Light minty | Minty |
| 3 months | 0.090% | 9% | 7.39 | Colorless clear water-thin liquid | Light minty | Minty |

(continued)

| Six (6) Months Accelerated Data (40°C/75%RH) for Oral Rinse 4 | | | | | | |
|---|---|---|---|---|---|---|
| Time Point | Stabilized $ClO_2$ (w/v) | Change of Stabilized $ClO_2$ from Day 0 Value | pH | Appearance/Color | Taste | Odor |
| 6 months | 0.089% | 10% | 7.25 | Colorless clear water-thin liquid | Light minty | Very faint minty |

**Table 9. Twelve (12) Month Ambient (25°C/60% Relative Humidity) Data for Oral Rinse 4**

| Twelve (12) Months Ambient Data (25°C/60%RH) for Oral Rinse 4 | | | | | | |
|---|---|---|---|---|---|---|
| Time Point | Stabilized $ClO_2$ (w/v) | Change of Stabilized $ClO_2$ from Day 0 Value | pH | Appearance/Color | Taste | Odor |
| Day 0 | 0.099% | Not Applicable | 7.22 | Colorless clear water-thin liquid | Light minty | Minty |
| 1 month | 0.098% | 1% | 7.55 | Colorless clear water-thin liquid | Light minty | Minty |
| 2 months | 0.095% | 4% | 7.41 | Colorless clear water-thin liquid | Light minty | Minty |
| 3 months | 0.094% | 5% | 7.43 | Colorless clear water-thin liquid | Light minty | Minty |
| 6 months | 0.093% | 6% | 7.40 | Colorless clear water-thin liquid | Light minty | Minty |
| 12 months | 0.093% | 6% | 7.25 | Colorless clear water-thin liquid | Very light minty | Light minty |

Oral Rinse 5 is a flavored, fluoride oral care composition containing the following ingredients: water, stabilized chlorine dioxide (0.099% w/v), trisodium phosphate, peppermint oil and polysorbate, sodium fluoride (0.052% w/w), and citric acid. (It should be noted that this formulation contains a very small amount of the humectant, propylene glycol.) Stability data for Oral Rinse 5, while stored under accelerated conditions, is presented in Table 10.

**Table 10. Three (3) Month Accelerated (40°C/75%RH) Data for Oral Rinse 5**

| Three (3) Months Accelerated Data (40°C/75%RH) for Oral Rinse 5 | | | | | | | | |
|---|---|---|---|---|---|---|---|---|
| Time Point | Stabilized $ClO_2$* (w/v) | Change of Stabilized $ClO_2$ from Day 0 Value | Sodium Fluoride* | Change of Sodium Fluoride from Day 0 Value | pH | Appearance/ Color | Taste | Odor |
| Day 0 | 0.099% | Not Applicable | 0.052% | Not Applicable | 7.26 | Colorless clear water-thin liquid | Light minty | Minty |
| 1 month | 0.093% | 6% | 0.051% | 2% | 7.54 | Colorless clear water-thin liquid | Light minty | Light minty |
| 2 months | 0.091% | 8% | Not Available (N/A) | N/A | 7.51 | Colorless clear water-thin liquid | Light minty | Light minty |
| 3 months | 0.090% | 9% | 0.049% | 6% | 7.43 | Colorless clear water-thin liquid | Light minty | Very light minty |

(continued)

| Three (3) Months Accelerated Data (40°C/75%RH) for Oral Rinse 5 | | | | | | | | |
|---|---|---|---|---|---|---|---|---|
| Time Point | Stabilized ClO$_2$* (w/v) | Change of Stabilized ClO$_2$ from Day 0 Value | Sodium Fluoride* | Change of Sodium Fluoride from Day 0 Value | pH | Appearance/ Color | Taste | Odor |
| *Average of two different samples being assayed. | | | | | | | | |

As illustrated in the stability data from Tables 8-10, Oral Rinse 4 and Oral Rinse 5 both demonstrate that the present invention provides improved stability for stabilized ClO$_2$ (the chlorine dioxide source) and pH over longer periods of time than those taught by US 6582682 (data shown in Tables 5-7). Unlike the tested prior art formulations (Oral Rinse 1, 2, 3), Oral Rinse 4 and Oral Rinse 5 maintains chemical stability (Criterion 1 and Criterion 2) and consumer goodness (Criterion 3, Criterion 4, and Criterion 5), as defined by the present invention, for either three (3) months while stored under accelerated conditions or twelve (12) months while stored under ambient conditions.

**Example 1: Method to make a Single-Phase Flavored Oral Rinse containing a Chlorine Dioxide Source**

**Step 1.** Flavoring System

Mix flavoring agent and emulsifier/suspender. Set mixture aside.

**Step 2.** Water Soluble Components

In a separate container, mix water, sweetener, phosphate buffer, and stabilized chlorine dioxide liquid.

**Step 3.** Final Phase

Combine step 1 mixture and step 2 mixture; mix thoroughly. Adjust pH of resulting mixture to pH 7.2 by adding a final pH adjuster (a weak organic acid such as acetic acid, fumaric acid, citric acid, or similar).
Note: If a fluoride ion source (such as sodium fluoride) is added to composition, this raw material would be added to the Water Soluble Components (Step 2).

**Example 2: Method to make a Single-Phase Flavored Oral Spray containing a Chlorine Dioxide Source**

**Step 1.** Flavoring System

Mix flavoring agent and emulsifier/suspender. Set mixture aside.

**Step 2.** Water Soluble Components

In a separate container, mix water, phosphate buffers (sodium phosphate monobasic and sodium phosphate dibasic), sweetener, and stabilized chlorine dioxide liquid. Phosphate buffers should be added in amounts that allow for a final pH of 6.8-7.0.

**Step 3.** Final Phase

Combine step 1 mixture and step 2 mixture; mix thoroughly.

**Step 4.** Packaging

Transfer the product from Step 3 into a suitable spray dispenser.

**Claims**

1. A flavored oral care single phase composition free of glycerin, castor oil and castor oil derivatives the composition having a pH in the range of 6.5 to 8.0 comprising:

   a) a source of chlorine dioxide;
   b) a flavoring system containing a flavoring agent emulsified in a polyoxyethylene sorbitan ester;
   c) a sweetener;
   d) a phosphate buffering system; and
   e) water.

2. The composition as set forth in Claim 1 wherein the composition is for releasing chlorine dioxide gas upon acidification of said composition in the oral cavity; and wherein said source of chlorine dioxide being stabilized in equilibrium with small amounts of free chlorine dioxide gas as demonstrated by not more than 10% degradation of the chlorine dioxide source within one year at ambient conditions or three months at 40°C and 75% relative humidity.

3. The composition as set forth in Claim 1 or Claim 2 wherein the concentration of said polyoxyethylene sorbitan ester in the final composition is in the range of about 0.001% to about 40.000% (w/w) for example in the range from about 0.01% to about 40.0% (w/w).

4. The composition as set forth in any preceding claim wherein the concentration of said flavoring agent in the final composition is in the range of about 0.005% to about 10.000% (w/w).

5. The composition as set forth in any preceding claim wherein the concentration of said sweetener in the final composition is in the range of about 0.005% to about 2.000% (w/w).

6. The composition as set forth in any preceding claim wherein said phosphate buffer maintains said composition at a pH in the range of about 6.5 to about 8.0, for example wherein the pH of said composition is maintained in the range of 7.3 to 7.5.

7. The composition as set forth in any preceding claim including a fluoride ion source, optionally wherein said fluoride ion source is selected from the group consisting of stannous fluoride, sodium fluoride, and sodium monofluorophosphate.

8. The composition as set forth in Claim 7 wherein the concentration of the fluoride ion source in the final composition is in the range of about 0.025% to about 0.080% (w/w), for example from about 0.080% to about 0.150% (w/w).

9. A method for making a single phase oral rinse containing a chlorine dioxide source, said method comprising the steps of:

   a) mixing a flavoring agent with an emulsifier comprising polyoxyethylene sorbitan ester;
   b) further mixing water, a sweetener, a phosphate buffer and the chlorine dioxide source;
   c) combining the mixtures obtained from exercise of said mixing and said further mixing;
   d) adjusting the pH of the mixture obtained by exercise of said step of combining;
   e) wherein said step of adjusting is carried out by the step of adding a weak organic acid to obtain a pH in the range of about 6.5 to about 8.0.

10. The method as set forth in Claim 9 wherein said step of adjusting by adding a weak organic acid is carried out to obtain a pH of 7.2; and/or wherein said step of adjusting is carried out by adding a weak organic acid selected from the group consisting of acetic acid, fumaric acid, and citric acid.

11. A method for making a single phase flavored oral spray; oral toothpaste or oral gel containing a chlorine dioxide source, said method comprising the steps of:

   a) mixing a flavoring agent and an emulsifier comprising polyoxyethylene sorbitan ester in a first container;
   b) further mixing water, a phosphate buffer, a sweetener, and a chlorine dioxide source liquid in a second container so as to provide a mixture having a pH in the range of 6.5 to 8.0;
   c) combining the mixtures in the first and second containers;

d) optionally adding excipient ingredients such as cellulose gum, hydrated silica, and titanium dioxide; and

e) transferring the combined mixtures into a spray dispenser or tube dispenser.

12. The method as set forth in any of claims 9 to 11 wherein said single phase flavored oral spray free of glycerin, castor oil and castor oil derivatives for preventing instability, and said chlorine dioxide source is for releasing chlorine dioxide gas upon acidification of the oral spray in the oral cavity.

13. The method as set forth in Claim 11 or Claim 12 including the step of selecting the phosphate buffer from a group consisting of sodium phosphate monobasic and sodium phosphate dibasic; and/or including the step of adjusting the pH of the combined mixtures to a pH in the range of about 6.5 to about 8.0, optionally wherein said step of adjusting is carried out by adding a quantity of the phosphate buffer.

14. A flavored oral care single phase composition free of glycerin, free of chlorate, chloride, chlorine, castor oil and castor oil derivatives, the composition having a pH in the range of 6.5 to 8.0 and said composition comprising:

a) a chlorine dioxide source capable of releasing chlorine dioxide gas upon acidification by amino acids normally found in human saliva within 10 seconds to 3 minutes and in equilibrium with a small amount of chlorine dioxide gas present in solution;

b) a phosphate buffer adapted for holding the composition at a neutral pH (6.0 to 7.2) in an aqueous solution;

c) a source of flavoring containing a polyoxyethylene sorbitan ester and a flavoring agent;

d) a sweetener;

e) optionally excipient ingredients such as cellulose gum, hydrated silica, and titanium dioxide; and

f) water.

## Patentansprüche

1. Aromatisierte einphasige Mundpflegezusammensetzung, die frei von Glycerin, Rizinusöl und Rizinusölderivaten ist, wobei die Zusammensetzung einen pH-Wert im Bereich von 6,5 bis 8,0 aufweist, umfassend:

a) eine Quelle von Chlordioxid;

b) ein Aromatisierungssystem, das ein in einem Polyoxyethylensorbitanester emulgiertes Aromatisierungsmittel enthält;

c) ein Süßungsmittel;

d) ein Phosphatpuffersystem; und

e) Wasser.

2. Zusammensetzung nach Anspruch 1, wobei die Zusammensetzung zum Freisetzen von Chlordioxidgas bei Ansäuerung der Zusammensetzung in der Mundhöhle dient; und wobei die Quelle von Chlordioxid im Gleichgewicht mit kleinen Mengen an freiem Chlordioxidgas stabilisiert ist, wie demonstriert durch nicht mehr als 10 % Abbau der Chlordioxidquelle innerhalb eines Jahres bei Umgebungsbedingungen oder drei Monaten bei 40 °C und 75 % relativer Feuchtigkeit.

3. Zusammensetzung nach Anspruch 1 oder Anspruch 2, wobei die Konzentration des Polyoxyethylensorbitanesters in der Endzusammensetzung im Bereich von etwa 0,001 % bis etwa 40,000 % (Gew./Gew.), beispielsweise im Bereich von etwa 0,01 % bis etwa 40,0 % (Gew./Gew.) liegt.

4. Zusammensetzung nach einem vorstehenden Anspruch, wobei die Konzentration des Aromatisierungsmittels in der Endzusammensetzung im Bereich von etwa 0,005 % bis etwa 10,000 % (Gew./Gew.) liegt.

5. Zusammensetzung nach einem vorstehenden Anspruch, wobei die Konzentration des Süßungsmittels in der Endzusammensetzung im Bereich von etwa 0,005 % bis etwa 2,000 % (Gew./Gew.) liegt.

6. Zusammensetzung nach einem vorstehenden Anspruch, wobei der Phosphatpuffer die Zusammensetzung auf einem pH-Wert im Bereich von etwa 6,5 bis etwa 8,0 hält, beispielsweise wobei der pH-Wert der Zusammensetzung im Bereich von 7,3 bis 7,5 gehalten wird.

7. Zusammensetzung nach einem vorstehenden Anspruch, die eine Fluoridionenquelle beinhaltet, wobei die Fluoridionenquelle ausgewählt ist aus der Gruppe, bestehend aus Zinn(II)fluorid, Natriumfluorid und Natriummonofluorphosphat.

8. Zusammensetzung nach Anspruch 7, wobei die Konzentration der Fluoridionenquelle in der Endzusammensetzung im Bereich von etwa 0,025 % bis etwa 0,080 % (Gew./Gew.), beispielsweise von etwa 0,080 % bis etwa 0,150 % (Gew./Gew.) liegt.

9. Verfahren zum Herstellen einer einphasigen Mundspülung, die eine Chlordioxidquelle enthält, wobei das Verfahren die folgenden Schritte umfasst:

   a) Mischen eines Aromatisierungsmittels mit einem Emulgator, der Polyoxyethylensorbitanester umfasst;
   b) weiter Mischen von Wasser, einem Süßungsmittel, einem Phosphatpuffer und der Chlordioxidquelle;
   c) Kombinieren der Mischungen, die aus der Ausübung des Mischens und des weiteren Mischens erhalten werden;
   d) Einstellen des pH-Werts der Mischung, die durch die Ausübung des Schritts des Kombinierens erhalten wird;
   e) wobei der Schritt des Einstellens durch den Schritt des Zugebens einer schwachen organischen Säure durchgeführt wird, um einen pH-Wert im Bereich von etwa 6,5 bis etwa 8,0 zu erhalten.

10. Verfahren nach Anspruch 9, wobei der Schritt des Einstellens durch Zugeben einer schwachen organischen Säure durchgeführt wird, um einen pH-Wert von 7,2 zu erhalten; und/oder wobei der Schritt des Einstellens durch Zugeben einer schwachen organischen Säure ausgewählt aus der Gruppe, bestehend aus Essigsäure, Fumarsäure und Citronensäure durchgeführt wird.

11. Verfahren zum Herstellen eines einphasigen aromatisierten Mundsprays; einer oralen Zahnpasta oder eines oralen Gels, das/die eine Chlordioxidquelle enthält, wobei das Verfahren die folgenden Schritte umfasst:

   a) Mischen eines Aromatisierungsmittels und eines Emulgators, der Polyoxyethylensorbitanester umfasst, in einem ersten Behälter;
   b) weiter Mischen von Wasser, einem Phosphatpuffer, einem Süßungsmittel und einer Chlordioxid-Quellenflüssigkeit in einem zweiten Behälter, um eine Mischung mit einem pH-Wert im Bereich von 6,5 bis 8,0 bereitzustellen;
   c) Kombinieren der Mischungen in dem ersten und zweiten Behälter;
   d) gegebenenfalls Zugeben von Hilfsstoffbestandteilen wie Cellulosegummi, hydratisiertem Siliciumdioxid und Titandioxid; und
   e) Überführen der kombinierten Mischungen in einen Sprühspender oder Tubenspender.

12. Verfahren nach einem der Ansprüche 9 bis 11, wobei das einphasige aromatisierte Mundspray frei von Glycerin, Rizinusöl und Rizinusölderivaten ist, um Instabilität zu verhindern, und die Chlordioxidquelle zum Freisetzen von Chlordioxidgas bei Ansäuerung des Mundsprays in der Mundhöhle dient.

13. Verfahren nach Anspruch 11 oder Anspruch 12, beinhaltend den Schritt des Auswählens des Phosphatpuffers aus einer Gruppe, bestehend aus monobasischem Natriumphosphat und dibasischem Natriumphosphat; und/oder beinhaltend den Schritt des Einstellens des pH-Werts der kombinierten Mischungen auf einen pH-Wert im Bereich von etwa 6,5 bis etwa 8,0, wobei gegebenenfalls der Schritt des Einstellens durch Zugeben einer Menge des Phosphatpuffers durchgeführt wird.

14. Aromatisierte einphasige Mundpflegezusammensetzung, die frei von Glycerin, frei von Chlorat, Chlorid, Chlor, Rizinusöl und Rizinusölderivaten ist, wobei die Zusammensetzung einen pH-Wert im Bereich von 6,5 bis 8,0 aufweist, und wobei die Zusammensetzung Folgendes umfasst:

   a) eine Chlordioxidquelle, die in der Lage ist, bei Ansäuerung durch Aminosäuren, die normalerweise im menschlichen Speichel vorkommen, innerhalb von 10 Sekunden bis 3 Minuten Chlordioxidgas freizusetzen und im Gleichgewicht mit einer kleinen Menge an in Lösung vorhandenem Chlordioxidgas vorliegt;
   b) einen Phosphatpuffer, der zum Halten der Zusammensetzung auf einem neutralen pH-Wert (6,0 bis 7,2) in einer wässrigen Lösung eingestellt ist;
   c) eine Aromatisierungsquelle, die einen Polyoxyethylensorbitanester und ein Aromatisierungsmittel enthält;
   d) ein Süßungsmittel;
   e) gegebenenfalls Hilfsstoffbestandteile wie Cellulosegummi, hydratisiertes Siliciumdioxid und Titandioxid; und

f) Wasser.

## Revendications

1. Composition monophasique aromatisée de soins bucco-dentaires exempte de glycérine, d'huile de ricin et de dérivés d'huile de ricin, la composition ayant un pH dans la plage de 6,5 à 8,0, comprenant :

   a) une source de dioxyde de chlore ;
   b) un système d'aromatisation contenant un agent aromatisant émulsifié dans un ester de polyoxyéthylène sorbitan ;
   c) un édulcorant ;
   d) un système de tampon phosphate ; et
   e) de l'eau.

2. Composition selon la revendication 1, dans laquelle la composition est destinée à libérer du dioxyde de chlore gazeux lors d'une acidification de ladite composition dans la cavité buccale ; et dans laquelle ladite source de dioxyde de chlore est stabilisée à l'équilibre avec de petites quantités de dioxyde de chlore gazeux libre comme démontré par pas plus de 10 % de dégradation de la source de dioxyde de chlore en un an dans des conditions ambiantes ou trois mois à 40 °C et 75 % d'humidité relative.

3. Composition selon la revendication 1 ou la revendication 2, dans laquelle la concentration dudit ester de polyoxyéthylène sorbitan dans la composition finale est dans la plage d'environ 0,001 % à environ 40,000 % (en poids) par exemple, dans la plage d'environ 0,01 % à environ 40,0 % (en poids).

4. Composition selon l'une quelconque revendication précédente, dans laquelle la concentration dudit agent aromatisant dans la composition finale est dans la plage d'environ 0,005 % à environ 10,000 % (en poids).

5. Composition selon l'une quelconque revendication précédente, dans laquelle la concentration dudit édulcorant dans la composition finale est dans la plage d'environ 0,005 % à environ 2,000 % (en poids).

6. Composition selon l'une quelconque revendication précédente, dans laquelle ledit tampon phosphate maintient ladite composition à un pH dans la plage d'environ 6,5 à environ 8,0, par exemple, dans laquelle le pH de ladite composition est maintenu dans la plage de 7,3 à 7,5.

7. Composition selon l'une quelconque revendication précédente, incluant une source d'ions fluorure, éventuellement dans laquelle ladite source d'ions fluorure est choisie dans le groupe constitué de fluorure stanneux, fluorure de sodium et monofluorophosphate de sodium.

8. Composition selon la revendication 7, dans laquelle la concentration de la source d'ions fluorure dans la composition finale est dans la plage d'environ 0,025 % à environ 0,080 % (en poids), par exemple, d'environ 0,080 % à environ 0,150 % (en poids).

9. Procédé de fabrication d'un produit de rinçage buccal monophasique contenant une source de dioxyde de chlore, ledit procédé comprenant les étapes de :

   a) mélange d'un agent aromatisant avec un émulsifiant comprenant un ester de polyoxyéthylène sorbitan ;
   b) mélange supplémentaire d'eau, d'un édulcorant, d'un tampon phosphate et de la source de dioxyde de chlore ;
   c) combinaison des mélanges obtenus par la réalisation dudit mélange et dudit mélange supplémentaire ;
   d) ajustement du pH du mélange obtenu par la réalisation de ladite étape de combinaison ;
   e) dans lequel ladite étape d'ajustement est effectuée par l'étape d'ajout d'un acide organique faible pour obtenir un pH dans la plage d'environ 6,5 à environ 8,0.

10. Procédé selon la revendication 9, dans lequel ladite étape d'ajustement par ajout d'un acide organique faible est effectuée pour obtenir un pH de 7,2 ; et/ou dans lequel ladite étape d'ajustement est effectuée par ajout d'un acide organique faible choisi dans le groupe constitué d'acide acétique, acide fumarique et acide citrique.

11. Procédé de fabrication d'un spray buccal aromatisé monophasique ; d'une pâte dentifrice buccale ou d'un gel buccal contenant une source de dioxyde de chlore, ledit procédé comprenant les étapes de :

a) mélange d'un agent aromatisant et d'un émulsifiant comprenant un ester de polyoxyéthylène sorbitan dans un premier récipient ;

b) mélange supplémentaire d'eau, d'un tampon phosphate, d'un édulcorant et d'une source de dioxyde de chlore liquide dans un deuxième récipient de façon à fournir un mélange ayant un pH dans la plage de 6,5 à 8,0 ;

c) combinaison des mélanges dans les premier et deuxième récipients ;

d) ajout éventuel d'ingrédients excipients tels que de la gomme de cellulose, de la silice hydratée et du dioxyde de titane ; et

e) transfert des mélanges combinés dans un atomiseur ou distributeur en tube.

12. Procédé selon l'une quelconque des revendications 9 à 11, dans lequel ledit spray buccal aromatisé monophasique est exempt de glycérine, d'huile de ricin et de dérivés d'huile de ricin pour empêcher une instabilité, et ladite source de dioxyde de chlore sert à libérer du dioxyde de chlore gazeux lors d'une acidification du spray buccal dans la cavité buccale.

13. Procédé selon la revendication 11 ou la revendication 12, incluant l'étape de sélection du tampon phosphate dans un groupe constitué de phosphate de sodium monobasique et de phosphate de sodium dibasique ; et/ou incluant l'étape d'ajustement du pH des mélanges combinés à un pH dans la plage d'environ 6,5 à environ 8,0, éventuellement dans lequel ladite étape d'ajustement est effectuée en ajoutant une quantité du tampon phosphate.

14. Composition monophasique aromatisée de soins bucco-dentaires exempte de glycérine, exempte de chlorate, de chlorure, de chlore, d'huile de ricin et de dérivés d'huile de ricin, la composition ayant un pH dans la plage de 6,5 à 8,0 et ladite composition comprenant :

a) une source de dioxyde de chlore capable de libérer du dioxyde de chlore gazeux lors d'une acidification par des acides aminés normalement trouvés dans la salive humaine dans les 10 secondes à 3 minutes et en équilibre avec une petite quantité de dioxyde de chlore gazeux présent en solution ;

b) un tampon phosphate conçu pour maintenir la composition à un pH neutre (6,0 à 7,2) dans une solution aqueuse ;

c) une source d'aromatisation contenant un ester de polyoxyéthylène sorbitan et un agent aromatisant ;

d) un édulcorant ;

e) éventuellement des ingrédients excipients tels que de la gomme de cellulose, de la silice hydratée et du dioxyde de titane ; et

f) de l'eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4330531 A
- US 5616347 A
- US 6039934 A, Alliger
- US 4861514 A
- US 4689215 A
- US 4696811 A
- US 4786492 A
- US 4788053 A
- US 4792442 A
- US 4793989 A
- US 4818519 A
- US 4851213 A
- US 4855135 A
- US 4886657 A
- US 4889714 A
- US 4925656 A
- US 5200171 A
- US 5348734 A
- US 5489435 A
- US 5618550 A

- US 5811115 A
- US 5834003 A
- US 5902575 A
- US 5935592 A
- US 6017554 A
- US 6077502 A
- US 6132702 A
- US 6235269 B
- US 6251372 B
- US 6264924 B
- US 6350438 B
- US 6696047 B
- US 6582682 B
- US 6696047 A
- US 20050196370 A, Yu
- US 20080269353 A
- US 2003129144 A
- EP 0613678 A
- US 2010221198 A

### Non-patent literature cited in the description

- *EPA,* 1999